# EUROPÄISCHE PATENTSCHRIFT

(11) **EP 2 438 872 B1**
(45) Veröffentlichungstag und Bekanntmachung des Hinweises auf die Patenterteilung: **04.11.2020**
(21) Anmeldenummer: 11180153.6
(22) Anmeldetag: 06.09.2011
(51) Int. Cl.: A61F 2/915, A61F 2/91, A61B 17/70, A61B 17/88

(54) **Medizinisches Implantat, insbesondere Stent, zur Implantation in einen tierischen und/oder menschlichen Körper**
Medical implant, in particular a stent, for implantation in an animal body and/or human body
Implant médical, notamment stent, pour l'implantation dans un corps animal et/ou humain

(30) Priorität: 08.10.2010 US 391085 P
(43) Veröffentlichungstag der Anmeldung: 11.04.2012
(73) Patentinhaber: Biotronik AG, 8180 Bülach (CH)
(72) Erfinder: Mews, Steffen, 18057 Rostock (DE); Bakczewitz, Frank, 18055 Rostock (DE)
(74) Vertreter: Galander, Marcus

(56) Entgegenhaltungen:
- EP-A1- 1 557 138
- WO-A1-2010/103344
- WO-A2-98/53760
- WO-A2-2004/045450
- WO-A2-2007/131002
- WO-A2-2010/011699
- DE-A1- 19 728 337

## Beschreibung

Die Erfindung betrifft ein medizinisches Implantat, insbesondere einen Stent, zur Implantation in einen tierischen und/oder menschlichen Körper nach dem Oberbegriff des Patentanspruchs 1.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprothesen oder Stents.

Aus der US 2009/0248136 A1 ist ein Stent bekannt, der mäanderförmige Streben aufweist, bei dem es beim Dilatieren des Stents mittels eines teleskopierbaren Ratschenmechanismus zur irreversiblen Veränderung einer Länge der jeweils angegriffenen Strebe kommt.
EP 1 557 138 offenbart einen Stent mit mindestens zwei Abschnitten einer Stentstruktur, die beim Expandieren aufeinander zu oder voneinander weg bewegt werden. Dabei ist zwischen den beiden Abschnitten eine Koppeleinrichtung angeordnet.

DE 197 28 337 offenbart eine Gefäßstütze, die biologisch abbaubar ist und auf unterschiedliche Durchmesser einstellbar ist. Die Gefäßstütze ist im aufgeweiteten Zustand stabilisiert.

WO 98/53760 offenbart einen Stent, der axialer Kompression widersteht.

WO 2004/045450 offenbart eine Endoprothese mit einem Einführzustand und einem entfalteten Zustand. Die Endoprothese umfasst eine gewebte rohrförmige Struktur und Mittel zur Aufrechterhaltung des expandierten Zustands auf.

WO 2010/103344 offenbart ein expandierbares Implantat, welches in einen Wirbelkörper implantiert werden kann.

WO 2010/011699 offenbart eine endoluminale Stützstruktur, welche über Drehgelenke verbundene Streben aufweist, so dass eine Serie verbundener Scherenmechanismen gebildet wird.

Der Erfindung liegt die Aufgabe zugrunde, ein Implantat bereitzustellen, das exakt dilatiert werden kann und zugleich eine hohe Stabilität aufweist.

Die Aufgabe wird erfindungsgemäß durch die Merkmale des Patentanspruchs 1 gelöst. Günstige Ausgestaltungen und Vorteile der Erfindung ergeben sich aus den weiteren Ansprüchen, der Zeichnung und der Beschreibung.

Die Erfindung geht aus von einem medizinischen Implantat, insbesondere einem Stent, zur Implantation im tierischen und/oder menschlichen Körper mit einem Grundkörper, der zumindest zwei Stege mit zumindest zwei sich in axialer Richtung gegenüberliegenden Umlenkstellen und zumindest ein an die Umlenkstellen angreifendes Verstellmittel zum Verstellen einer Erstreckung des Grundkörpers in Umfangrichtung aufweist.

Es wird vorgeschlagen, dass mittels des Verstellmittels ein axialer Abstand zwischen den zwei Umlenkstellen verkürzbar ist. Durch die erfindungsgemäße Ausgestaltung kann vorteilhaft ein Implantat bereitgestellt werden, bei dem mittels eines Verstellmittels Kosten und Bauraum sparend eine Expansion des Implantats mit einer großen Radialkraft und einer hohen Steifigkeit des voll expandierten Implantats zur fixen Verankerung einer Zielform des Implantats erreicht wird. Hierdurch kann Gewebe schonend eine Überdilatation einer Implantationsstelle oder eines Gefäßes verhindert werden. Ferner kann die Implantation Material schonend durchgeführt werden, was zu einem geringen Ausfall des Implantats und folglich zu einem komplikationslosen Eingriff führt.

In diesem Zusammenhang soll unter einem "Implantat" insbesondere ein Körper verstanden werden, der zumindest eine Ersatzfunktion permanent oder für einen längeren Zeitraum bei Implantation in einen tierischen und/oder menschlichen Körper erfüllt. Denkbar wären hier sämtliche, dem Fachmann als zweckdienlich erscheinenden medizinischen Implantate, wie beispielsweise eine Gefäßprothese oder besonders vorteilhaft wird eine Ausbildung des medizinischen Implantats als ein Stent. Durch die Ausführung des Implantats als Stent bzw. dadurch dass der Grundkörper einen Stent umfasst, kann eine konstruktiv einfach zu implantierende Struktur bereitgestellt werden.

Ferner soll in diesem Zusammenhang unter einem "Grundkörper" insbesondere eine Struktur, wie beispielsweise ein Drahtgeflecht, verstanden werden, die im Wesentlichen eine Gestalt und/oder eine Form des Implantats bzw. insbesondere eine Form des Stents und/oder den Stent selbst bildet. Zudem ist der Grundkörper vorzugsweise aus einem metallischen Material und/oder aus einer Kombination von mehreren metallischen Materialien gefertigt, wie beispielsweise Eisen, Magnesium, Nickel, Wolfram, Titan, Zirkonium, Niob, Tantal, Zink, Silizium, Lithium, Natrium, Kalium, Kalzium, Mangan und/oder jedem anderen, dem Fachmann als sinnvoll erscheinenden Material. Möglich wären auch eine Zink-Kalziumlegierung und/oder ein Formgedächtnis-Material, wie beispielsweise eine Kupfer-Zink-Aluminium-Legierung und/oder Nickel-Titan-Legierung, vorzugsweise Nitinol. Alternativ kann es vorteilhaft sein, wenn der Grundkörper zumindest Kobalt und/oder Chrom aufweist, vorzugsweise in der Form von Edelstahl und/oder eines Cr-Ni-Fe-Stahl - hier bevorzugt die Legierung 316L - oder einer Co-Cr-Legierung. Durch diese Ausgestaltung kann ein Implantat bereitgestellt werden, das eine gute Dilatierbarkeit und eine vorteilhafte Flexibilität gepaart mit einer hohen Stabilität aufweist. Grundsätzlich wäre es jedoch auch denkbar, dass der Grundkörper des Implantats mindestens teilweise aus Kunststoff, einer Keramik und/oder aus einem biodegradierbaren Material besteht.

Des Weiteren soll unter einem "Steg" eine Struktur des Grundkörpers verstanden werden, die sich in Umfangsrichtung des Implantats erstreckt und eine Mäander- bzw. Wellenform aufweist und von einem Stentstrut gebildet sein kann. In diesem Zusammenhang soll unter einer "Umlenkstelle" eine Stelle des Stegs verstanden werden, an dem sich eine Orientierung einer Verlaufsrichtung des Stegs ändert. Vorteilhafterweise stellt die Umlenkstelle ein Minimum oder ein Maximum in der Erstreckung des Stegs in axialer Richtung des Implantats dar. Hierbei können Maxima, die sich in Umfangsrichtung auf gleicher Höhe befindende bzw. sich in axialer Richtung gegenüberliegen und zu axial benachbart angeordneten Stegen gehören, beide Maxima oder beide Minima oder Maxima und Minima sein. Bevorzugt liegt jedoch ein Minimum einem Maximum gegenüber und besonders bevorzugt sind die Minima eines ersten Stegs mit den Maxima eines zweiten Stegs, insbesondere stoffschlüssig, verbunden und bilden eine Kontaktstelle. Dadurch ist der Grundkörper aus Zellen aufgebaut. Diese Zellen können jede, dem Fachmann als zweckdienlich erscheinende Form, wie rund, oval, rechteckig und/oder Rautenform aufweisen. Diese Form ist insbesondere dazu ausgebildet, faltbar zu sein. Besonders bevorzugt sind die Zellen im Wesentlichen rautenförmig ausgestaltet. Unter der Wendung "im Wesentlichen rautenförmig" soll hier insbesondere verstanden werden, dass auch Formen, die einer Raute bzw. einem Rhombus ähnlich sind, wie beispielsweise eine rautenähnliche Form mit abgerundeten Ecken und/oder konkaven und/oder konvexen Seiten unter dem Begriff "rautenförmig" verstanden werden sollen. Die Minima und Maxima bilden im zusammengefalteten Zustand des Implantats bevorzugt je einen spitzen Winkel.

Unter einem "Verstellmittel" soll in diesem Zusammenhang ein Mittel verstanden werden, mittels dem zumindest ein Parameter, wie eine Erstreckung des Grundkörpers in Umfangrichtung und damit einen Querschnitt einer Fläche senkrecht zur axialen Richtung des Grundkörpers und eine axiale Länge des Grundkörpers verstellt wird. Unter der Wendung "an die Umlenkstellen angreifend" soll hier verstanden werden, dass Kräfte insbesondere Verstellkräfte vom Verstellmittel, insbesondere über die Stege, auf die Umlenkstellen übertragen werden und/oder dass das Verstellmittel, insbesondere über die Stege, in direktem Kontakt zu den Umlenkstellen stehen. Das Verstellmittel stellt hier nicht ein Expansionsmittel in der Form eines Ballonkatheters und/oder eine Eigenschaft einer Selbstexpansion eines Implantats, insbesondere mittels eines Formgedächtnismaterials, dar. Mittels der erfindungsgemäßen Verkürzung des axialen Abstands zwischen den Umlenkstellen durch das Verstellmittel ist auch der Grundkörper in seiner axialen Länge verkürzbar. Dies kann beispielsweise mittels der rautenförmigen Zellen konstruktiv besonders einfach erfolgen.

Des Weiteren wird vorgeschlagen, dass das Verstellmittel ein zweites Expansionsmittel darstellt und ein erstes Expansionsmittel vorgesehen ist, das separat von dem zweiten Expansionsmittel anwendbar ist. In diesem Zusammenhang soll unter einem Expansionsmittel insbesondere ein Mittel verstanden werden, das das Implantat bzw. den Grundkörper expandiert, also den Querschnitt der Fläche senkrecht zu der axialen Richtung des Grundkörpers vergrößert. Ferner soll unter einem "ersten Expansionsmittel" insbesondere ein Ballonkatheters und/oder eine Eigenschaft einer Selbstexpansion des Implantats, insbesondere mittels eines Formgedächtnismaterials, und/oder jedes andere, dem Fachmann für zweckdienlich scheinende Mittel verstanden werden. Unter "anwendbar" soll hier insbesondere betreibbar, gestaltbar und/oder ausführbar verstanden werden, wobei die Wendung "separat anwendbar" hier insbesondere meint, dass die beiden Expansionsmittel von unterschiedlichen Bauteilen gebildet sind und/oder mit unterschiedlichen Expansionsmechanismen betrieben werden und/oder dass das der Expansionsmechanismus des zweiten Expansionsmittel unabhängig von der Expansion des Grundkörpers durch das erste Expansionsmittel ist. Ferner soll unter "vorgesehen" speziell ausgestattet, ausgelegt, ausgebildet und/oder vorbereitet verstanden werden. Mittels der beiden Expansionsmittel können vorteilhaft unterschiedliche Expansionsmechanismen angewendet werden, was zu einem besonders vielseitig einsetzbaren Implantat führt.

Vorteilhafterweise ist das zweite Expansionsmittel dazu ausgebildet, nach dem ersten Expansionsmittel expandierbar zu sein. Unter der Wendung "zeitlich nach" soll insbesondere nachträglich verstanden werden. Durch die Realisierung eines Zeitversatzes kann eine nach der ersten Expansion eingestellte Positionierung vorteilhaft verfeinert und fixiert werden. Somit dient das Verstellmittel bzw. das zweite Expansionsmittel einer Fixierung einer expandierten Form bzw. der Zielform des Grundkörpers.

Nach einer Aufweitung ballonexpandierbarer Stents tritt im Allgemeinen ein Rückstoß bzw. eine elastische Rückfederung (Recoil) des Stents auf, dessen Ausgleich zu einer Überdilatation der Implantationsstelle führen kann. Demnach wird in einer weiteren Ausgestaltung der Erfindung vorgesehen, dass das Verstellmittel eine starre Länge aufweist, sodass ein Rückstoß bei der Expansion des ersten Expansionsmittels unterbindbar ist. In diesem Zusammenhang soll unter einer "starren Länge" insbesondere eine unänderbare und/oder fixierte Länge, insbesondere in axialer Richtung des Verstellmittels, und unter "unterbindbar" reduzierbar und/oder vorteilhaft verhinderbar verstanden werden. Durch die Realisierung der Verhinderung des Rückstoßes kann zum einen die Implantationsstelle geschont werden und zum anderen kann eine Materialbeanspruchung reduziert werden, was zu einer höheren Sicherheit des Implantates führt. Ferner kann eine Position des Implantats genau an eine Dimension der Implantationsstelle angepasst werden, was wiederum in einem sicheren und exakten Sitz resultiert.

Ferner ist es vorteilhaft, wenn sich das Verstellmittel in axialer Richtung entlang einer axialen Länge des Grundkörpers erstreckt. Hierbei erstreckt sich das Verstellmittel bevorzugt entlang der gesamten axialen Länge des Grundkörpers. Durch diese Ausgestaltung kann eine Funktionsweise des Verstellmittels konstruktiv einfach mit einer Konstruktion des Implantats kombiniert werden.

Ferner wird vorgeschlagen, dass das Implantat zumindest eine Durchführung hat, die an zumindest einer Umlenkstelle angeordnet ist und für das Verstellmittel vorgesehen ist. Unter einer "Durchführung" soll hier insbesondere eine Ausnehmung mit einer glatten Innenwandung verstanden werden, die von dem Verstellmittel durchgriffen wird. Bevorzugt weisen hierfür zwei aneinender verbundene Umlenkstellen als Maximum und Minimum miteinander fluchtende Durchführungen auf. Grundsätzlich können entlang der axialen Länge des Verstellmittels mehrere von diesen durchgriffenen Durchführungen vorgesehen sein. Mittels der Durchführung kann das Verstellmittel konstruktiv einfach und störungsfrei in den Grundkörper integriert werden.

Zudem ist es vorteilhaft, wenn an den in axialer Richtung äußeren Stegen des Grundkörpers zueinander weisende Ausnehmungen, beispielsweise ausgeführt als Sacklöcher, zur Aufnahme von axialen Enden des Verstellmittels angeordnet sind, wodurch ein Überstand des Verstellmittels über die axiale Länge des Implantat respektive den Grundkörper verhindert werden kann, wodurch eine Reizung von umliegendem Gewebe minimiert werden kann.

Des Weiteren kann es vorteilhaft sein, wenn das Implantat zumindest zwei Verstellmittel aufweist, die in Umfangsrichtung über einen Umfang des Grundkörpers verteilt angeordnet sind, wodurch eine axiale Verkürzung des Grundkörpers vorteilhaft homogen über den Umfang des Implantats erfolgen kann, wodurch unvorteilhafte Schrägstellungen des Implantats am Implantationsstelle verhindert werden können. Ferner ist es vorteilhaft, wenn die Verstellmittel symmetrisch über den Umfang verteil angeordnet sind und zweckmäßigerweise parallel zueinander verlaufen, wodurch ein vom Verstellmittels vermittelter Bewegungsvorgang gleichförmig realisiert werden kann. Grundsätzlich kann eine Anzahl an Verstellmittel soweit erhöht werden, dass jede Kontaktstelle zwischen einem Maximum und einem Minimum eine Ausnehmung und/oder ein Paar an Durchführungen aufweist, was ein besonders gleichmäßiges Verstellen ermöglicht.

Eine einfache und unkomplizierte Funktion bzw. ein einfaches Betreiben des Verstellmittels kann vorteilhaft erreicht werden, wenn das Verstellmittelsmittel zumindest einen Aktuatoransatzpunkt für einen Aktuator aufweist. In diesem Zusammenhang soll unter einem "Aktuatoransatzpunkt" insbesondere eine Ausformung, wie eine Nase, ein Bolzen, ein Sechskantbolzen, ein Schlitz, ein Kreuzschlitz und/oder eine andere, dem Fachmann für sinnvoll erscheinende Ausformung, verstanden werden, in und/oder an der ein Aktuator, wie ein Bohrer, ein Schraubenschlüssel, eine Ratsche, eine Kurbel und/oder eine anderer, dem Fachmann für einsetzbar erscheinender Aktuator, angesetzt werden kann, um das Verstellmittel zu betreiben. Vorteilhafterweise ist der Aktuatoransatzpunkt für einen extern betätigbaren Aktuator vorgesehen, also ist der Aktuator vorzugsweise von außerhalb des Körpers betreibbar, wodurch das Verstellmittel besonders einfach betrieben werden kann. Der Aktuatoransatzpunkt ist an einem axial nach außen weisenden Ende des Verstellmittels angeordnet. Die Ausnehmung des axial äußeren Stegs, der an einem Ende des Grundkörpers angeordnet ist, das entgegen einer Implantationsrichtung weist, ist eine durchgehende Ausnehmung und wird von dem Ende des Verstellmittels mit dem Aktuatoransatzpunkt so durchgriffen, dass das Verstellmittel bündig mit dem axialen Ende des Grundkörpers bzw. einer Ausnehmungen axialen Außenfläche des ersten Stegs endet.

Zudem wird vorgeschlagen, dass das Verstellmittel zumindest zwei miteinander verschraubbare Bauteile aufweist. Diese Bauteile weisen bevorzugt komplementäre Gewinde auf und sind in den axial äußeren Stegen des Grundkörpers fixiert. Vorteilhafterweise ist ein erstes Bauteil mit einem Innengewinde ausgeführt und/oder als Zylinder ausgebildet, der in der Ausnehmung befestigbar oder einschraubbar ist, die in dem äußeren Steg angeordnet ist, der sich an einem Ende des Grundkörpers befindet, das in die Implantationsrichtung weist. Ein zweites Bauteil, bevorzugt ausgebildet als eine die Durchführung(en) durchspannende Stange, ist in der Ausnehmung fixiert, die in dem axial äußeren Steg in dem entgegen der Implantationsrichtung weisenden Ende des Grundkörpers angeordnet ist. Die Fixierung erfolgt so, dass eine Rotationsbewegung stattfinden kann. Ferner weist das zweite Bauteil an einem dem Ende mit dem Aktuatoransatzpunkt gegenüberliegenden Ende ein Außengewinde auf. Bei einem Verstellen des Verstellmittels werden die beiden Bauteile so miteinander verschraubt, dass sich ihre addierte Gesamtlänge und die Länge der Stentzellen verkürzen. Durch die erfindungsgemäße Ausgestaltung kann das Verstellmittel konstruktiv einfach realisiert und zuverlässig gestaltet werden.

Grundsätzlich wäre es auch möglich, dass das Bauteil mit Innengewinde ein Innengewinde selbst ist, das in der Ausnehmung angeformt ist, die in dem axial äußeren Steg angeordnet ist, der sich an dem in Implantationsrichtung weisenden Ende des Grundkörpers befindet. Hierbei ist die Ausnehmung zur Limitierung einer Schraubtiefe als Sackloch ausgeführt. Prinzipiell wäre es jedoch auch denkbar, dass die Durchführung(en) und die Ausnehmung des axial äußeren Stegs, der an dem entgegen der Implantationsrichtung weisenden Ende des Grundkörpers angeordnet ist, mit Innengewinden ausgeführt sind und eine Gewindestange mit diesen zur axialen Abstandsverkürzung verschraubt wird. Hierbei müsste jedoch zum Schutz des Umfelds zum Implantat ein Mittel zur Abdeckung des Überstands der Gewindestange über die Ausnehmung bereitgestellt werden.

Eine bevorzugte Weiterbildung besteht darin, dass zumindest eines der Bauteile zumindest einen gewindefreien Abschnitt zur Zentrierung der miteinander verschraubbaren Bauteile bei der Expansion durch das erste Expansionsmittel aufweist. Zweckmäßigerweise ist der gewindefreie Abschnitt an dem ersten Bauteil angeordnet und als glatte Zylinderhülse ausgebildet. Vorteilhafterweise greift das zweite Bauteil mit seinem das Außengewinde tragenden Ende im unexpandierten Zustand des Implantats in den gewindefreien Abschnitt ein. Dadurch können während der ersten Expansion, beispielsweise mittels eines Ballonkatheters, die beiden Bauteile verliersicher übereinander gleiten, wodurch die für das Verschrauben benötigten Positionen der beiden Bauteile prozesssicher eingestellt werden können.

Eine weitere Ausgestaltung der Erfindung sieht vor, dass das Verstellmittelsmittel einen Durchmesser von zumindest 200 µm, bevorzugt zumindest 400 µm und besonders vorteilhaft von zumindest 500 µm aufweist. Somit ist das Verstellmittel vorteilhaft auf die Dimensionen des Implantats mit beispielsweise Stentstruts mit einer Wandstärke von ca. 600 µm angepasst und es kann zudem eine stabile Struktur bereitgestellt werden.

Des Weiteren wird vorgeschlagen, dass das Verstellmittelsmittel ein erstes Bauteil und zumindest ein zweites Bauteil aufweist, wobei die Bauteile als verdrillbare Drähte ausgeführt sind, wodurch ein preiswertes, gewichtsarmes und unkomplexes Verstellmittel erreicht wird.

Es wird zudem vorgeschlagen, dass der Grundkörper einen Stabilisierungskörper für einen Knochen, insbesondere einen Wirbelkörper umfasst. Bevorzugt ist das Implantat als Wirbelkörperstent (vertebral body stent) ausgeführt. Durch diese erfindungsgemäße Ausgestaltung kann ein gut einsetzbares und zuverlässiges Implantat für ein Einsatzgebiet realisiert werden, in dem besonders formstabile und robuste Implantate benötigt werden.
Die Erfindung ist nachfolgend beispielhaft, anhand von in Zeichnungen dargestellten Ausführungsbeispielen, näher erläutert. Es zeigen:
- Fig. 1: ein erfindungsgemäßes Implantat in einer perspektivischen Ansicht mit einer Aktuatorvorrichtung,
- Fig. 2: eine Detailansicht des Implantats aus Fig. 1 vor der Expansion mittels des Verstellmittels,
- Fig. 3: eine Detailansicht des Implantats aus Fig. 1 nach der Expansion mittels des Verstellmittels,
- Fig. 4: eine schematische Darstellung des Implantats an einer Implantationsstelle und
- Fig. 5: eine Detailansicht einer alternativen Ausführung des Implantats.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Zur Vermeidung unnötiger Wiederholungen wird bei nicht näher beschriebenen Elementen in einer Figur auf die jeweilige Beschreibung der Elemente in vorstehenden Figuren verweisen.

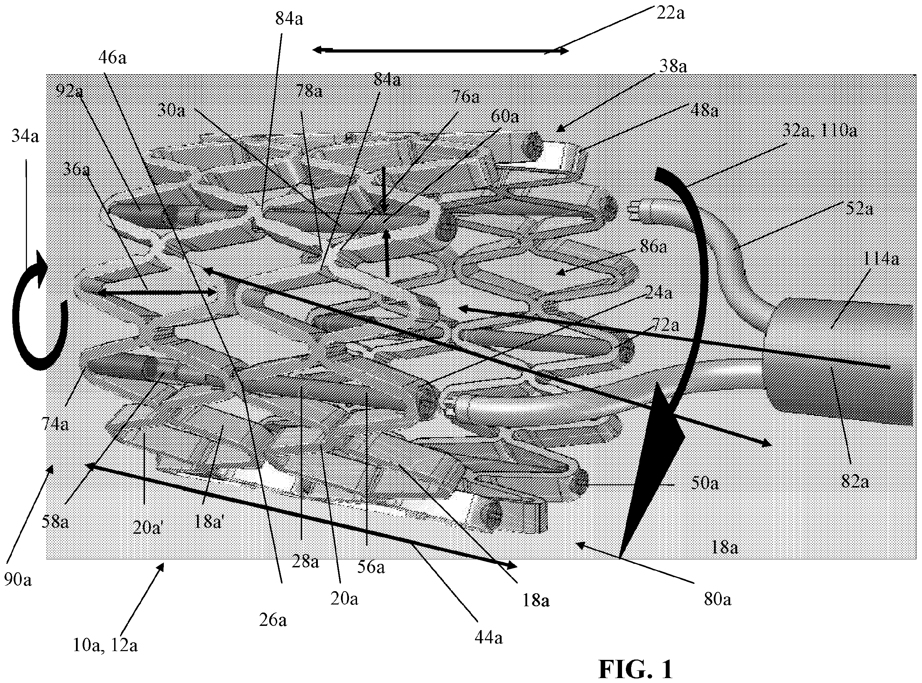

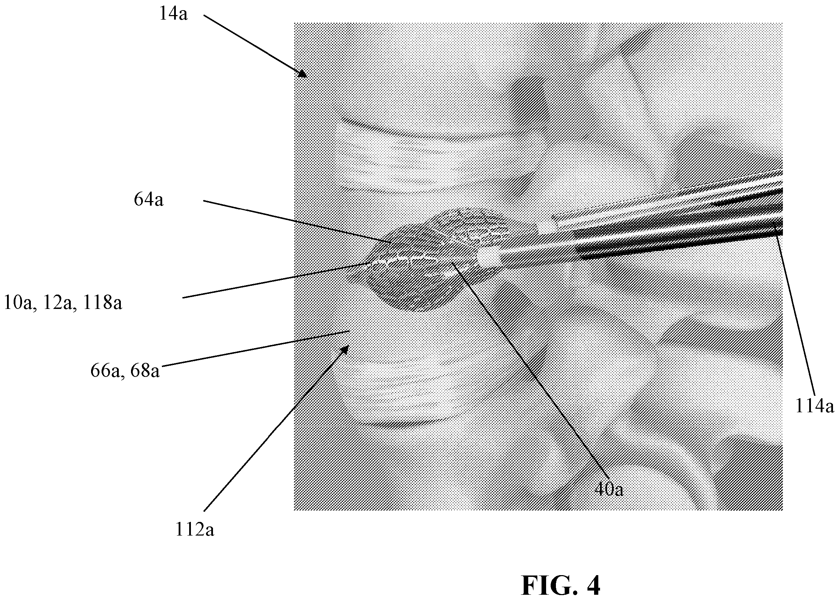

Fig. 1 zeigt in einer perspektivischen Ansicht ein medizinisches Implantat 10a ausgeführt als Stent12a zur Implantation in einen tierischen und/oder menschlichen Körper 14a (siehe Fig. 4), mit einem Grundkörper 16a, der den Stent 12a umfasst und als Material einen medizinischen Edelstahl bzw. eine Chrom-Kobalt-Legierung aufweist. Der Grundkörper 16a weist als Grundkörperstruktur ein Drahtgeflecht auf, das in einer dem Fachmann bekannter Weise von mäanderförmigen Stegen 18a, 18a', 20a, 20a' bzw. Stentstruts 70a gebildet ist. Die Stege 18a, 18a', 20a, 20a' erstrecken sich in einer Umfangsrichtung 34a entlang eines Umfangs 48a des Grundkörpers 16a und sind in einer axialen Richtung 22a hintereinander angeordnet. Jeder Steg 18a, 18a', 20a, 20a' weist über seinen Verlauf entlang des Umfangs 48a abwechselnd Minima 72a und Maxima 74a auf, die jeweils eine Umlenkstelle 24a, 26a, 76a, 78a bilden. Hierbei liegen jeweils Maxima 74a des Stegs 18a, der einen ersten Steg bildet und der an einem Ende 80a des Grundkörpers 16a angeordnet ist, das entgegen einer Implantationsrichtung 82a weist, und Minima 72a des Stegs 20a, der einen zweiten Steg bildet, sich in axialer Richtung 22a gegenüber, wodurch sich ebenso die Umlenkstellen 24a, 26a gegenüber liegen. Die Minima 72a des ersten Stegs 18a und die Maxima 74a des zweiten Stegs 20a stoßen in axialer Richtung 22a aneinander an und bilden jeweils eine Kontaktstelle 84a, in der die Stege 18a, 20a stoffschlüssig verbunden sind. Gleiche Kontaktstellen 84a sind auch zwischen den Minima 72a des zweiten Stegs 20a und Maxima 74a eines dritten Stegs 18a' gebildet. Grundsätzlich kann sich diese Struktur in axialer Richtung 22a beliebig weit fortsetzen, hier sind jedoch nur vier Stege 18a, 18a', 20a, 20a' gezeigt.

Durch diesen Aufbau weist das Drahtgeflecht bzw. der Grundkörper 16a eine Vielzahl von Zellen 86a auf, die im Wesentlichen rautenförmig ausgestaltet sind und in Unfangsrichtung 34a nebeneinander und in axialer Richtung 22a hintereinander angeordnet sind. Hierbei bilden im gefalteten Zustand des Implantats 10a die Minima 72a bzw. die Maxima 74a und somit die Umlenkstellen 24a, 26a spitze Winkel 88a der Rauten. Durch diese Ausgestaltung kann das Implantat 10a gefaltet werden und bei einer Expansion weiten sich die Zellen 86a in Umfangsrichtung 34a auf, wobei sich ihre Erstreckung in axialer Richtung 22a verkürzt.

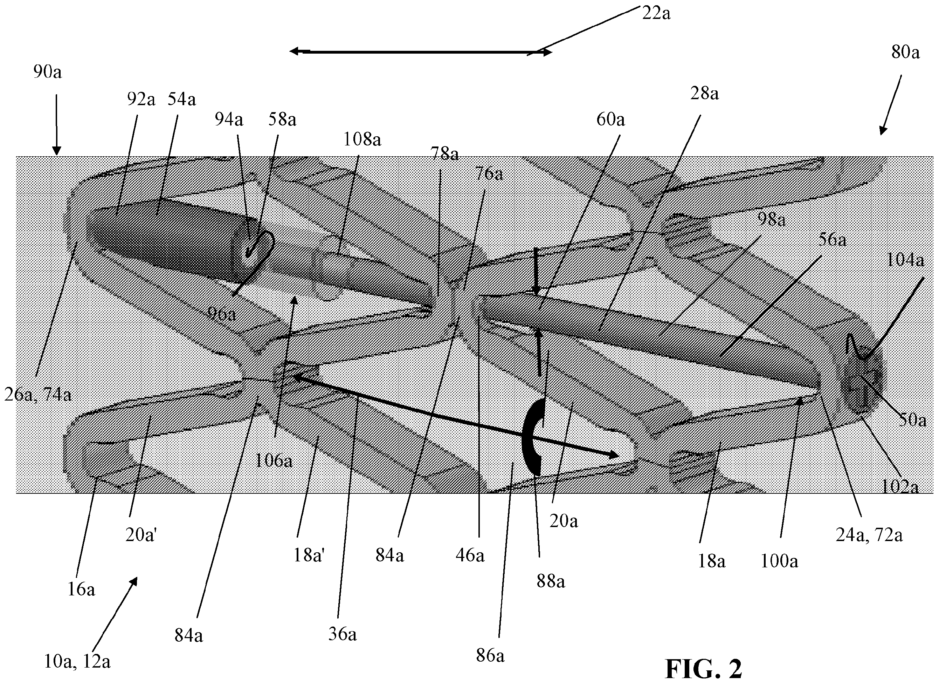

Zudem weist der Grundkörper 16a mehrere an die Umlenkstellen 24a, 26a angreifende Verstellmittel 28a, 30a zum Verstellen einer Erstreckung 32a des Grundkörpers 16a in Umfangrichtung 34a auf. Die Verstellmittel 28a, 30a sind in Umfangsrichtung 34a über den Umfang 48a des Grundkörpers 16a verteilt bzw. hier sind sieben Verstellmittel 28a, 30a mit jeweils einem Abstand von ca. 50° über den Umfang 48a regelmäßig verteilt und parallel zueinander angeordnet. Ferner erstrecken die Verstellmittel 28a, 30a in axialer Richtung 22a entlang einer axialen Länge 44a des Grundkörpers 16a und sind in axialer Richtung 22a gleich lang wie der Grundkörper 16a von einem Maximum 74a des ersten Stegs 18a und einem Minimum 72a des vierten Stegs 20a', der an einem Ende 90a des Grundkörpers 16a angeordnet ist, das in Implantationsrichtung 82a weist. Ferner weisen die Verstellmittelsmittel 28a, 30a einen Durchmesser 60a von 500 µm auf und sind somit in einer gleichen Größenordnung wie Wandstärken der Stentstruts 70a, die ca. 600 µm ist. Des Weiteren weist der Grundkörper 16a des Implantats 10a Durchführungen 46a auf, die an den Umlenkstellen 24a, 26a angeordnet sind und für das Verstellmittel 28a, 30a vorgesehen sind (vgl. auch Fig. 2). Die Durchführungen 46a sind in die Kontaktstellen 84a zwischen den Minima 72a des zweiten Stegs 20a und den Maxima 74a des dritten Stegs 18a' als miteinander fluchtende Ausnehmungen mit glatten Innenoberflächen eingebracht. Die Verstellmittel 28a, 30a weisen je zwei miteinander verschraubbare Bauteile 54a; 56a auf, wobei das erste Bauteil 54a von einem Zylinder 92a gebildet ist, der in einem Sackloch 94a ein Innengewinde 96a aufweist. Der Zylinder 92a ist an der Umlenkstelle 26a angeordnet und zwar an einer entgegen der Implantationsrichtung 82a weisenden Seite eines Minimums 72a des vierten Stegs 20a' und ist beispielsweise mittels einem nicht dargestellten Bolzen in einer hier nicht gezeigten Ausnehmung der Minima 72a, befestigt. Zudem ist an dem ersten Bauteil 54a ein gewindefreier Abschnitt 58a, in der Form eines glatten Zylinderhülsenabschnitt, zur Zentrierung der miteinander verschraubbaren Bauteile 54a, 56a bei der Expansion durch ein erstes Expansionsmittel 38a angeformt bzw. ausgeführt.

Das zweite Bauteil 56a ist als eine die Durchführung 46a durchspannende Stange 98a ausgebildet und ist mit einem Endabschnitt 100a in einer Ausnehmung 102a fixiert, die die Umlenkungen 24a bzw. die Maxima 74a des Stegs 18a durchspannt. Hierbei schließt der Endabschnitt 100a bündig mit dem Grundkörper 16a bzw. einer axialen Außenfläche 104a des Stegs 18a ab. Ferner weist das zweite Bauteil 56a an einem dem Endabschnitt 100a gegenüberliegenden Ende 106a ein mit dem Innengewinde 96a des ersten Bauteils 54a korrespondierendes Außengewinde 108a auf. An dem Endabschnitt 100a der Stange 98a bzw. der Verstellmittelsmittel 28a, 30a ist je ein entgegen der Implantationsrichtung 82a weisender Aktuatoransatzpunkt 50a, in der Form eines Kreuzschnitzes, für einen Aktuator 52a bzw. einen extern betätigbaren Aktuator 52a, ausgeführt als Kurbel 110a, angeformt.

Die Verstellmittel 28a, 30a stellen ein zweites Expansionsmittel 38a dar, mittels dem das Implantat 10a nach einer Vorpositionierung an einer Implantationsstelle 112a positioniert wird (vgl. Fig. 4). Die Vordilatation bzw. Vorpositionierung erfolgt mittels eines separat von dem zweiten Expansionsmittel 38a anwendbaren ersten Expansionsmittels 40a in der Form eines nicht dargestellten Ballonkatheters. Die Verstellmittel 28a, 30a bzw. das zweite Expansionsmittel 38a ist somit dazu ausgebildet, nach dem ersten Expansionsmittel 40a expandierbar zu sein.

In der Fig. 2 ist ein Ausschnitt des Implantats 10a mit einem Verstellmittel 28a im Zustand nach der Expansion mit dem ersten Expansionsmittel 40a gezeigt. Hierbei ist das Ende 106a mit dem Außengewinde 108a des zweiten Bauteils 56a in dem gewindefreien Abschnitt 58a des ersten Bauteils 54a angeordnet bzw. zentriert. Dies erfolgte bei der Dilatation mit dem ersten Expansionsmittels 40a, da diese Anteile 58a, 108a übereinander gleiten und ineinander geschoben werden können.

Wird nun, wie in der Fig. 1 angedeutet, mit dem Aktuator 52a bzw. der Kurbel 110a mit zwei Armen, der/die durch eine Implantationsvorrichtung 114a in der Form von einer Kanüle, zum Stent 12a geführt wird, der Aktuatorpunkt 50a betätigt, dreht sich das Außengewinde 108a in das Innengewinde 96a des Sacklochs 94a des ersten Bauteils 52a. Dies wird für jedes Verstellmittel 28a, 30a bzw. für jedes Paar an Verstellmittels 28a, 30 durchgeführt. Das Verschrauben erfolgt so lange bis entweder der Stent 12a seine gewünschte Position erreicht hat oder das Ende 106a der Stange 98a an dem Sackloch 94a anschlägt. Ist dies erreicht wird die Kurbel 110a durch die Kanüle abgezogen.

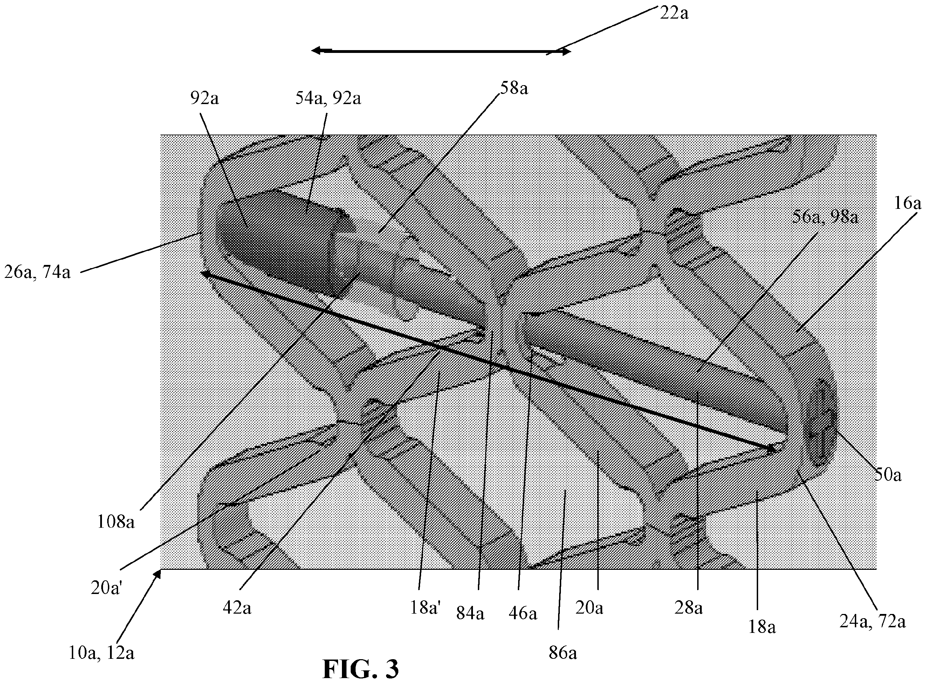

Fig. 3 zeigt denselben Abschnitt des Implantats 10a wie Fig. 2 nach der Expansion mittels des zweiten Expansionsmittels 38a. Durch das Verschrauben der Bauteil 54a, 56a mittels des Verstellmittels 28a, 30a nimmt eine gemeinsame Länge der Bauteile 54a, 56a ab, wodurch ein axialer Abstand 36a zwischen den zwei Umlenkstellen 24a, 26a verkürzt wird. Dadurch werden die Zellen 86a durch die die Verstellmittel 28a, 30a verlaufen gezwungen sich in Umfangsrichtung 34a zu öffnen. Hiervon werden auch benachbarte verstellmittellose Zellen 86a beeinflusst, die ebenfalls ihre Rautenform ändern. Hierdurch kann beispielsweise auch eine Quader ähnliche Stentform provoziert werden (nicht gezeigt).

Ferner weisen die Verstellmittel 28a, 30a durch die fest verschraubten Bauteil 54a, 56a eine starre Länge 42a zur Fixierung des Stents 12a in seiner Endposition auf, sodass ein Rückstoß bei der Expansion des ersten Expansionsmittels 40a unterbindbar ist bzw. nicht zum tragen kommt, da die Endposition des Stents12a mittels der Verstellmittels 28a, 30a rückstoßfrei eingestellt werden kann. Somit ermöglichen die Verstellmittel 28a, 30a eine Aufweitung eines Querschnitts 116a einer Fläche des Grundkörpers 16a, die senkrecht zur axialen Richtung 22a des Grundkörpers 16a angeordnet ist, und damit eine Anpassung der Stentform an Gegebenheiten bzw. eine Dimension der Implantationsstelle 112a ohne ein Umfeld der Implantationsstelle 112a unnötig durch einen Rückstoß zu stressen.

Der Stent 12a ist als Wirbelkörperstent 118a (vertebral bosdy stent) ausgebildet und wird in einen kollabierten Wirbelkörper 68a implantiert. Die Fig. 4 zeigt schematisch die Implantationsstelle 112a mit einem implantierten Implantat 10a und der Implantationsvorrichtung 114a. Ist der Stent 12a an der gewollten Position fixiert, wird er auszementiert und kann so den defekten Wirbelkörper 68a stabilisieren, wodurch der Grundkörper 16a einen Stabilisierungskörper 64a für einen Knochen 66a bzw. den Wirbelkörper 68a umfasst. Hierbei wäre beispielsweise die Einnahme der Quaderform des Stents 12a zur Erhöhung der Stabilität des Stents 12a vorteilhaft.

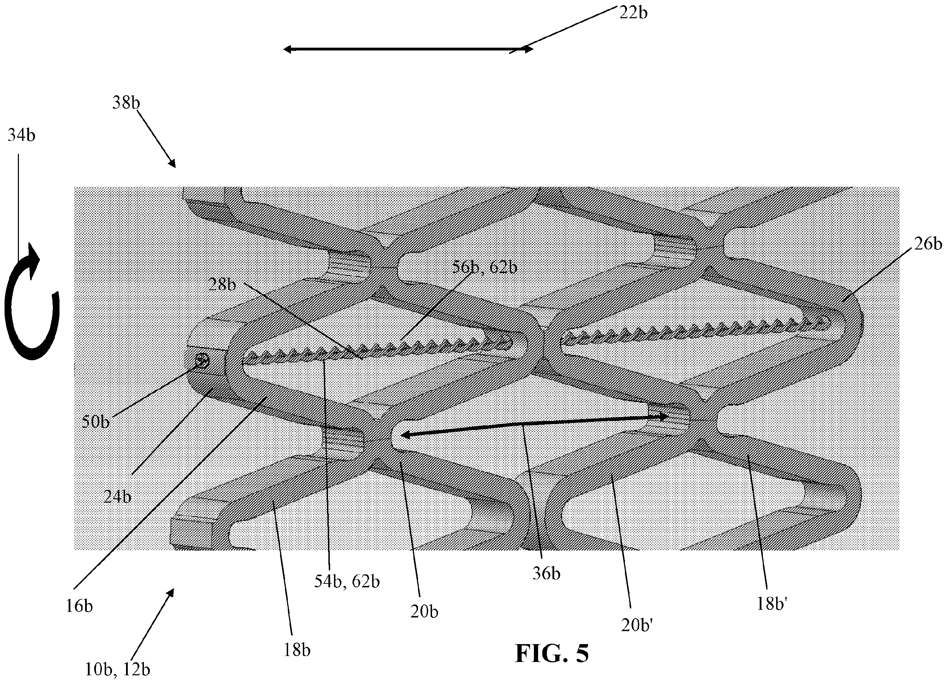

In der Figur 5 ist ein alternatives Ausführungsbeispiel des medizinischen Implantats 10a dargestellt. Im Wesentlichen sind gleich bleibende Bauteile, Merkmale und Funktionen grundsätzlich mit den gleichen Bezugszeichen beziffert. Zur Unterscheidung der Ausführungsbeispiele sind jedoch den Bezugszeichen der Ausführungsbeispiele die Buchstaben a und b hinzugefügt. Die nachfolgende Beschreibung beschränkt sich im Wesentlichen auf die Unterschiede zu dem Ausführungsbeispiel in den Figuren 1 bis 4, wobei bezüglich gleich bleibender Bauteile, Merkmale und Funktionen auf die Beschreibung des Ausführungsbeispiels in den Figuren 1 bis 6 verwiesen werden kann.

In Figur 5 ist ein Ausschnitt eines alternativen medizinischen Implantats 10b in der Form eines Stents 12b bzw. eines Wirbelkörperstents 118a mit einem Grundkörper 16b, der Stege 18b, 20b mit sich in axialer Richtung 22a gegenüberliegenden Umlenkstellen 24b, 26b und ein sich durch eine Durchführung 46b erstreckendes und an die Umlenkstellen 24b, 26b angreifendes Verstellmittel 28b aufweist. Das Verstellmittelsmittel 28b bzw. auch ein hier nicht gezeigtes zweites Verstellmittel 30b sind als zweites Expansionsmittel 38a ausgebildet und weisen ein erstes Bauteil 54b und ein zweites Bauteil 56b auf, wobei die Bauteile 54b, 56b als verdrillbare Drähte 62b ausgeführt sind. Durch das Verdrillen der Drähte 62b mittels eines hier nicht gezeigten Aktuators an einem Aktuatoransatzpunkt 50b wird ein axialer Abstand 36b zwischen den Umlenkstellen 24b, 26b verkürzt. Hierdurch wird eine Rautenstruktur einer Zelle 86b es Grundkörpers 16b verändert, wodurch einer Erstreckung 32b des Grundkörpers 16b in Umfangrichtung 34b verstellt bzw. vergrößert wird.

### Bezugszeichenliste

- 10: Implantat
- 12: Stent
- 14: Körper
- 16: Grundkörper
- 18: Steg
- 20: Steg
- 22: Richtung
- 24: Umlenkstelle
- 26: Umlenkstelle
- 28: Verstellmittel
- 30: Verstellmittel
- 32: Erstreckung
- 34: Umfangsrichtung
- 36: Abstand
- 38: Expansionsmittel
- 40: Expansionsmittel
- 42: Länge
- 44: Länge
- 46: Durchführung
- 48: Umfang
- 50: Aktuatoransatzpunkt
- 52: Aktuator
- 54: Bauteil
- 56: Bauteil
- 58: Abschnitt
- 60: Durchmesser
- 62: Draht
- 64: Stabilisierungskörper
- 66: Knochen
- 68: Wirbelkörper
- 70: Stentstrut
- 72: Maxima
- 74: Minima
- 76: Umlenkstelle
- 78: Umlenkstelle
- 80: Ende
- 82: Implantationsrichtung
- 84: Kontaktstelle
- 86: Zelle
- 88: Winkel
- 90: Ende
- 92: Zylinder
- 94: Sackloch
- 96: Innengewinde
- 98: Stange
- 100: Endabschnitt
- 102: Ausnehmung
- 104: Außenfläche
- 106: Ende
- 108: Außengewinde
- 110: Kurbel
- 112: Implantationsstelle
- 114: Implantationsvorrichtung
- 116: Querschnitt
- 118: Wirbelkörperstent

## Patentansprüche

1. Medizinisches Implantat (10a, 10b), insbesondere Stent (12a, 12b), zur Implantation im tierischen und/oder menschlichen Körper (14a, 14b) mit einem Grundkörper (16a, 16b), der zumindest zwei Stege (18a, 18a', 18b, 18b'; 20a, 20a', 20b, 20b') mit zumindest zwei sich in axialer Richtung (22a, 22b) gegenüberliegenden Umlenkstellen (24a, 24b; 26a, 26b) und zumindest ein an die Umlenkstellen (24a, 24b; 26a, 26b) angreifendes Verstellmittel (28a, 28b; 30a, 30b) zum Verstellen einer Erstreckung (32a, 32b) des Grundkörpers (16a, 16b) in Umfangrichtung (34a, 34b) aufweist, **dadurch gekennzeichnet, dass** sich an der jeweiligen Umlenkstelle (24a, 24b; 26a, 26b) eine Orientierung einer Verlaufsrichtung des jeweiligen Steges (18a, 18a', 18b, 18b'; 20a, 20a', 20b, 20b') ändert, wobei ein Steg eine Struktur des Grundkörpers ist, die sich in Umfangsrichtung des Implantats erstreckt und eine Mäander- bzw. Wellenform aufweist,
wobei mittels des Verstellmittels (28a, 28b; 30a, 30b) ein axialer Abstand (36a, 36b) zwischen den zwei Umlenkstellen (24a, 24b; 26a, 26b) verkürzbar ist.

2. Medizinisches Implantat nach Anspruch 1, **dadurch gekennzeichnet, dass** das Verstellmittel (28a, 28b; 30a, 30b) ein zweites Expansionsmittel (38a, 38b) darstellt und ein erstes Expansionsmittel (40a, 40b) vorgesehen ist, das separat von dem zweiten Expansionsmittel (38a, 38b) anwendbar ist.

3. Medizinisches Implantat nach Anspruch 1 oder 2, **dadurch gekennzeichnet, dass** das Verstellmittel (28a, 28b; 30a, 30b) eine starre Länge (42a, 42b) aufweist, sodass ein Rückstoß bei der Expansion des ersten Expansionsmittels (40a, 40b) unterbindbar ist.

4. Medizinisches Implantat nach einem der vorhergehenden Ansprüche, **dadurch gekennzeichnet, dass** sich das Verstellmittel (28a, 28b; 30a, 30b) in axialer Richtung (22a, 22b) entlang einer axialen Länge (44a, 44b) des Grundkörpers (16a, 16b) erstreckt.

5. Medizinisches Implantat nach einem der vorhergehenden Ansprüche, **gekennzeichnet durch** zumindest eine Durchführung (46a, 46b), die an zumindest einer Umlenkstelle (24a, 24b; 26a, 26b) angeordnet ist und für das Verstellmittel (28a, 28b; 30a, 30b) vorgesehen ist.

6. Medizinisches Implantat nach einem der vorhergehenden Ansprüche, **gekennzeichnet durch** zumindest zwei Verstellmittel (28a, 28b; 30a, 30b), die in Umfangsrichtung (34a, 34b) über einen Umfang (48a, 48b) des Grundkörpers (16a, 16b) verteilt angeordnet sind.

7. Medizinisches Implantat nach einem der vorhergehenden Ansprüche, **dadurch gekennzeichnet, dass** das Verstellmittelsmittel (28a, 28b; 30a, 30b) zumindest einen Aktuatoransatzpunkt (50a, 50b) für einen Aktuator (52a, 52b) aufweist, vorzugsweise einen Aktuatoransatzpunkt (50a, 50b) für einen extern betätigbaren Aktuator (52a, 52b).

8. Medizinisches Implantat nach einem der vorhergehenden Ansprüche, **dadurch gekennzeichnet, dass** das Verstellmittel (28a, 28b; 30a, 30b) zumindest zwei miteinander verschraubbare Bauteile (54a; 56a) aufweist.

9. Medizinisches Implantat nach Anspruch 2 und 8, **dadurch gekennzeichnet, dass** zumindest eines der Bauteile (54a, 56a) zumindest einen gewindefreien Abschnitt (58a) zur Zentrierung der miteinander verschraubbaren Bauteile (54a, 56a) bei der Expansion durch das erste Expansionsmittel (38a) aufweist.

10. Medizinisches Implantat nach einem der vorhergehenden Ansprüche, **dadurch gekennzeichnet, dass** das Verstellmittelsmittel (28a, 28b; 30a, 30b) einen Durchmesser (60a, 60b) von zumindest 500 µm aufweist.

11. Medizinisches Implantat nach einem der vorhergehenden Ansprüche, **dadurch gekennzeichnet, dass** das Verstellmittelsmittel (28b, 30b) ein erstes Bauteil, (54b) und zumindest ein zweites Bauteil (56b) aufweist, wobei die Bauteile (54b, 56b) als verdrillbare Drähte (62b) ausgeführt sind.

12. Medizinisches Implantat nach einem der vorhergehenden Ansprüche, **dadurch gekennzeichnet, dass** der Grundkörper (16a, 16b) einen Stabilisierungskörper (64a, 64b) für einen Knochen (66a, 66b), insbesondere einen Wirbelkörper (68a, 68b) umfasst.

## Claims

1. A medical implant (10a, 10b), in particular a stent (12a, 12b), for implantation in an animal body and/or a human body (14a, 14b), comprising a base body (16a, 16b), which comprises at least two webs (18a, 18a', 18b, 18b'; 20a, 20a', 20b, 20b') having at least two deflection points (24a, 24b; 26a, 26b) located opposite one another in the axial direction (22a, 22b), and at least one adjusting means (28a, 28b; 30a, 30b) acting on the deflection points (24a, 24b; 26a, 26b) for adjusting an extension (32a, 32b) of the base body (16a, 16b) in the circumferential direction (34a, 34b), **characterized in that** an orientation of a direction of the respective stent (18a, 18a', 18b, 18b'; 20a, 20a', 20b, 20b') changes at the respective deflection point (24a, 24b; 26a, 26b), a web being a structure of the base body that extends in the circumferential direction of the implant and has a meander or wave shape, it being possible to shorten an axial distance (36a, 36b) between the two deflection points (24a, 24b; 26a, 26b) using the adjusting means (28a, 28b; 30a, 30b).

2. The medical implant according to claim 1, **characterized in that** the adjusting means (28a, 28b; 30a, 30b) represents a second expansion means (38a, 38b), and a first expansion means (40a, 40b) is provided, which can be used separately from the second expansion means (38a, 38b).

3. The medical implant according to claim 1 or 2, **characterized in that** the adjusting means (28a, 28b; 30a, 30b) has a rigid length (42a, 42b), whereby recoil can be prevented during the expansion of the first expansion means (40a, 40b).

4. The medical implant according to any one of the preceding claims, **characterized in that** the adjusting means (28a, 28b; 30a, 30b) extends in the axial direction (22a, 22b) along an axial length (44a, 44b) of the base body (16a, 16b).

5. The medical implant according to any one of the preceding claims, **characterized by** at least one passage (46a, 46b), which is provided at at least one deflection point (24a, 24b; 26a, 26b) and is intended for the adjusting means (28a, 28b; 30a, 30b).

6. The medical implant according to any one of the preceding claims, **characterized by** at least two adjusting means (28a, 28b; 30a, 30b), which are distributed in the circumferential direction (34a, 34b) across a circumference (48a, 48b) of the base body (16a, 16b).

7. The medical implant according to any one of the preceding claims, **characterized in that** the adjusting means (28a, 28b; 30a, 30b) includes at least one actuator engagement point (50a, 50b) for an actuator (52a, 52b), and preferably an actuator engagement point (50a, 50b) for an externally actuatable actuator (52a, 52b).

8. The medical implant according to any one of the preceding claims, **characterized in that** the adjusting means (28a, 28b; 30a, 30b) comprises at least two components (54a; 56a) that can be screwed together.

9. The medical implant according to claims 2 and 8, **characterized in that** at least one of the components (54a, 56a) comprises at least one non-threaded section (58a) for centering the components (54a, 56a) that can be screwed together during the expansion by the first expansion means (38a).

10. The medical implant according to any one of the preceding claims, **characterized in that** the adjusting means (28a, 28b; 30a, 30b) has a diameter (60a, 60b) of at least 500 µm.

11. The medical implant according to any one of the preceding claims, **characterized in that** the adjusting means (28b, 30b) comprises a first component (54b) and at least one second component (56b), the components (54b, 56b) being implemented as twistable wires (62b).

12. The medical implant according to any one of the preceding claims, **characterized in that** the base body (16a, 16b) comprises a stabilizing body (64a, 64b) for a bone (66a, 66b), and in particular a vertebral body (68a, 68b).

## Revendications

1. Implant médical (10a, 10b), notamment stent (12a, 12b), pour l'implantation dans un corps (14a, 14b) animal et/ou humain, avec un corps de base (16a, 16b) qui présente au moins deux branches (18a, 18a', 18b, 18b' ; 20a, 20a', 20b, 20b') avec au moins deux points de déviation (24a, 24b ; 26a, 26b) se situant l'un en face de l'autre en direction axiale (22a, 22b) et au moins un système de réglage (28a, 28b ; 30a, 30b) se mettant en prise au niveau des points de déviation (24a, 24b ; 26a, 26b) permettant de régler l'extension (32a, 32b) du corps de base (16a, 16b) dans la direction circonférentielle (34a, 34b),
**caractérisé en ce**
**qu'**au niveau du point de déviation (24a, 24b ; 26a, 26b) respectif, une orientation d'une direction d'extension de la branche (18a, 18a', 18b, 18b' ; 20a, 20a', 20b, 20b') respective se modifie, où une branche est une structure du corps de base qui s'étend dans la direction circonférentielle de l'implant et présente une forme en méandres, respectivement ondulée,
où une distance axiale (36a, 36b) entre les deux points de déviation (24a, 24b ; 26a, 26b) peut être raccourcie au moyen du système de réglage (28a, 28b ; 30a, 30b).

2. Implant médical selon la revendication 1, **caractérisé en ce que** le système de réglage (28a, 28b ; 30a, 30b) représente un deuxième système d'expansion (38a, 38b) et un premier système d'expansion (40a, 40b) est prévu qui peut être employé de manière séparée par rapport au deuxième système d'expansion (38a, 38b).

3. Implant médical selon la revendication 1 ou la revendication 2, **caractérisé en ce que** le système de réglage (28a, 28b ; 30a, 30b) présente une longueur rigide (42a, 42b) de sorte qu'un recul lors de l'expansion du premier système d'expansion (40a, 40b) peut être évité.

4. Implant médical selon l'une des revendications précédentes, **caractérisé en ce que** le système de réglage (28a, 28b ; 30a, 30b) s'étend en direction axiale (22a, 22b) le long d'une étendue axiale (44a, 44b) du corps de base (16a, 16b).

5. Implant médical selon l'une des revendications précédentes, **caractérisé par** au moins un passage (46a, 46b) qui est disposé au niveau d'au moins un point de déviation (24a, 24b ; 26a, 26b) et est prévu pour le système de réglage (28a, 28b ; 30a, 30b).

6. Implant médical selon l'une des revendications précédentes, **caractérisé par** au moins deux systèmes de réglage (28a, 28b ; 30a, 30b) qui sont disposés distribués sur un pourtour (48a, 48b) du corps de base (16a, 16b).

7. Implant médical selon l'une des revendications précédentes, **caractérisé en ce que** le système de réglage (28a, 28b ; 30a, 30b) présente au moins un point d'ancrage d'actionneur (50a, 50b) pour un actionneur (52a, 52b), de préférence un point d'ancrage d'actionneur (50a, 50b) pour un actionneur (52a, 52b) pouvant être actionné de manière extérieure.

8. Implant médical selon l'une des revendications précédentes, **caractérisé en ce que** le système de réglage (28a, 28b ; 30a, 30b) présente au moins deux composants (54a ; 56a) pouvant être vissés ensemble.

9. Implant médical selon la revendication 2 et la revendication 8, **caractérisé en ce qu'**au moins un des composants (54a, 56a) présente au moins un segment exempt de filetage (58a) permettant le centrage des composants (54a, 56a) pouvant être vissés ensemble lors de l'expansion par le premier système d'expansion (38a).

10. Implant médical selon l'une des revendications précédentes, **caractérisé en ce que** le système de réglage (28a, 28b ; 30a, 30b) présente un diamètre (60a, 60b) d'au moins 500 µm.

11. Implant médical selon l'une des revendications précédentes, **caractérisé en ce que** le système de réglage (28b, 30b) présente un premier composant (54b) et au moins un deuxième composant (56b), où les composants (54b, 56b) sont conçus sous forme de fils pouvant être torsadés (62b).

12. Implant médical selon l'une des revendications précédentes, **caractérisé en ce que** le corps de base (16a, 16b) comprend un corps de stabilisation (64a, 64b) pour un os (66a, 66b), notamment un corps vertébral (68a, 68b).
